# EUROPEAN PATENT APPLICATION

(11) **EP 4 287 201 A1**
(43) Date of publication of application: **06.12.2023**
(21) Application number: 22176135.6
(22) Date of filing: 30.05.2022
(51) Int. Cl.: G16H 30/40, G16H 50/30

(54) **COMPENSATING FOR DIFFERENCES IN MEDICAL IMAGES**

(71) Applicant: Koninklijke Philips N.V., 5656 AG Eindhoven (NL)
(72) Inventor: GRAß, Michael, Eindhoven (NL); BUELOW, Thomas, Eindhoven (NL); KROENKE, Sven, 5656AG Eindhoven (NL); VON BERG, Jens, 5656AG Eindhoven (NL); HARDER, Tim Philipp, Eindhoven (NL); SAALBACH, Axel, Eindhoven (NL); GOOßEN, André, Eindhoven (NL)
(74) Representative: Philips Intellectual Property & Standards

(57) **Abstract**

A computer-implemented method of compensating for differences in medical images, is provided. The method includes receiving (S110) image data comprising a temporal series of medical images (110_{1..i}). The temporal series includes one or more medical images generated by a first type of imaging modality (120), and one or more medical images generated by a second type of imaging modality (130, 130'). The first type of imaging modality is different to the second type of imaging modality. In one aspect, the method includes generating (S120a, S120b), from the temporal series of medical images (110_{1..i}), a normalised temporal series of medical images (140_{1..i}), and outputting (S130a, S130a', S130b) the normalised temporal series of medical images (140_{1..i}) and/or one or more measurement values derived therefrom. In another aspect, the method includes generating (S120a, S120b), from the temporal series of medical images (110_{1..i}), one or more normalised measurement values (150_{1..j}) representing a region of interest in the temporal series of medical images (110_{1..i}), and outputting the normalised measurement value(s) (150_{1..j}).

## Description

### FIELD OF THE INVENTION

The present disclosure relates to compensating for differences in medical images. A computer-implemented method, a computer program product, and a system, are disclosed.

### BACKGROUND OF THE INVENTION

Clinical investigations often involve the acquisition of medical images of a subject. The images may be acquired at different points in time, i.e. as a temporal series of images. The images may be used to identify temporal changes in a region of interest, and consequently to assess the progression of a medical condition in the subject. However, during the course of the clinical investigation, images may be acquired using different types of imaging modalities. For instance, factors such as the availability of imaging systems, the need to perform additional investigations on the subject's medical condition, and so forth, can result in medical images of the region of interest being acquired using different types of imaging modalities over time. The images generated by different types of imaging modalities have very different appearance. It is therefore difficult to accurately identify temporal changes in a region of interest from such images. This hampers the assessment of the progression of the medical condition in the subject.

By way of an example, a clinical investigation may be performed by generating projection X-ray images of a region of interest in a subject at different points in time. However, due to the availability of imaging systems, or due to the need to perform additional investigations, some of the images generated during the course of the clinical investigation may be generated using a computed tomography "CT" imaging system, or a magnetic resonance "MR" imaging system. Such images are very different in appearance to X-ray projection images, and there is also no direct correspondence between their image intensities and the image intensities in the X-ray projection images. Consequently, an assessment of the progression of a medical condition in the subject may be performed exclusively with images that are generated by a single type of imaging modality, even though data exists at additional points in time from a different type of imaging modality. This represents a missed opportunity, because the data at the additional points in time might otherwise provide valuable information for assessing the progression of the medical condition.

In addition to the challenge presented by images being acquired using different types of imaging modalities during the course of a clinical investigation, there may also be differences in the manner in which the images are acquired. For example, there may be differences in the subject's posture, or differences in the viewing angle of a medical imaging system, or differences in the amount of ionising radiation dose used to acquire the images. Such factors may be present even when images are acquired from a single type of imaging modality. These factors exacerbate the challenge of identifying temporal changes in the region of interest, and can even lead to an erroneous diagnosis of a subject's condition. Some of these factors can be resolved by applying facility-wide imaging protocols. For example, some clinical settings permit the positioning of a subject in an optimal and also standardised manner with respect to an imaging system. Protocols may also be also set wherein imaging systems from a common modality use a standardised amount of radiation dose. The application of facility-wide imaging protocols can reduce the amount of inter-image variability arising from differences in the manner in which images are acquired. However, the use of such protocols limits flexibility. In some clinical settings, it may also be impractical, or even impossible to position a subject with limited mobility in an optimal and standardised manner with respect to the imaging system. For example in intensive care settings it may be impractical, or even impossible, to acquire images of a subject with a desired posture, state of inspiration, or with the subject in a desired position with respect to a medical imaging system. Consequently, differences in the manner in which the images are acquired can also hamper the assessment of the progression of the medical condition in the subject. In particular it is the fact that these confounding factors are not constant over time, and that they differ between each of the images, that makes it challenging to assess longitudinal changes in the subject's condition. As a result, a clinician interpreting such images may instinctively compensate for such differences, which can give rise to an erroneous diagnosis.

Consequently, there is a need for improvements that facilitate the identification of changes in a temporal series of medical images.

### SUMMARY OF THE INVENTION

According to one aspect of the present disclosure, a computer-implemented method of compensating for differences in medical images, is provided. The method includes:
receiving image data comprising a temporal series of medical images, the temporal series including one or more medical images generated by a first type of imaging modality, and one or more medical images generated by a second type of imaging modality, the first type of imaging modality being different to the second type of imaging modality;
generating, from the temporal series of medical images, a normalised temporal series of medical images, or one or more normalised measurement values representing a region of interest in the temporal series of medical images; and
outputting the normalised temporal series of medical images and/or one or more measurement values derived from the normalised temporal series of medical images, or outputting the one or more normalised measurement values, respectively.

In the above method, the normalised temporal series of medical images, the one or more measurement values derived from the normalised temporal series of medical images, and the one or more normalised measurement values, each provide compensation for differences in the type of imaging modality that is used to acquire the images in the temporal series. By providing such compensation, a more reliable comparison between the images can be made, which permits a more reliable assessment to be made of longitudinal changes in a medical condition in the subject.

Further aspects, features, and advantages of the present disclosure will become apparent from the following description of examples, which is made with reference to the accompanying drawings.

### BRIEF DESCRIPTION OF THE DRAWINGS

Fig. 1 is a flowchart illustrating an example of a method of compensating for differences in medical images, in accordance with some aspects of the present disclosure.
Fig. 2 is a schematic diagram illustrating an example of a system 200 for compensating for differences in medical images, in accordance with some aspects of the present disclosure.
Fig. 3 is a schematic diagram illustrating a first example of a method of compensating for differences in medical images, in accordance with some aspects of the present disclosure.
Fig. 4 is a schematic diagram illustrating an example of the operation of projecting a volumetric medical image 110₃ onto a virtual detector 240ᵥ using a virtual source 230ᵥ, in accordance with some aspects of the present disclosure.
Fig. 5 illustrates an example of an atlas image 170 for the lung field, in accordance with some aspects of the present disclosure.
Fig. 6 illustrates an example of an atlas image 170 for the rib cage, in accordance with some aspects of the present disclosure.
Fig. 7 illustrates an example of the result of applying feature detector to a medical image 140₁ to identify a plurality of landmarks in the medical image, in accordance with some aspects of the present disclosure.
Fig. 8 illustrates an example of the warping of two 2D medical images in a temporal series, 140₁, 140₂, to a reference image, in accordance with some aspects of the present disclosure.
Fig. 9 illustrates an example of the warping of two 2D medical images in a temporal series, 140₁, 140₂, to a reference image, and the adjustment of the intensity of the medical images, in accordance with some aspects of the present disclosure.
Fig. 10 illustrates an example of the suppression of image features outside a lung region of interest in medical image 140₁, in accordance with some aspects of the present disclosure.
Fig. 11 illustrates an example of the warping of two normalised medical images 140₁, 140₂, to a reference image, and the adjusting of image intensity values in the images, and the suppression of image features outside a lung region of interest, in accordance with some aspects of the present disclosure.
Fig. 12 is a schematic diagram illustrating a second example of a method of compensating for differences in medical images, in accordance with some aspects of the present disclosure.

### DETAILED DESCRIPTION OF EMBODIMENTS

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to computer-implemented methods that involve the processing of image data relating to a temporal series of medical images. In some examples, reference is made to medical images that represent the lungs of a subject. However, it is to be appreciated that the lungs serve only as an example, and that the methods disclosed herein may alternatively be used with medical images that represent any portion of the anatomy.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact diskread only memory "CD-ROM", compact disk-read/write "CD-R/W", Blu-Ray^{™} and DVD.
As mentioned above, there is a need for improvements that facilitate the identification of changes in a temporal series of medical images.

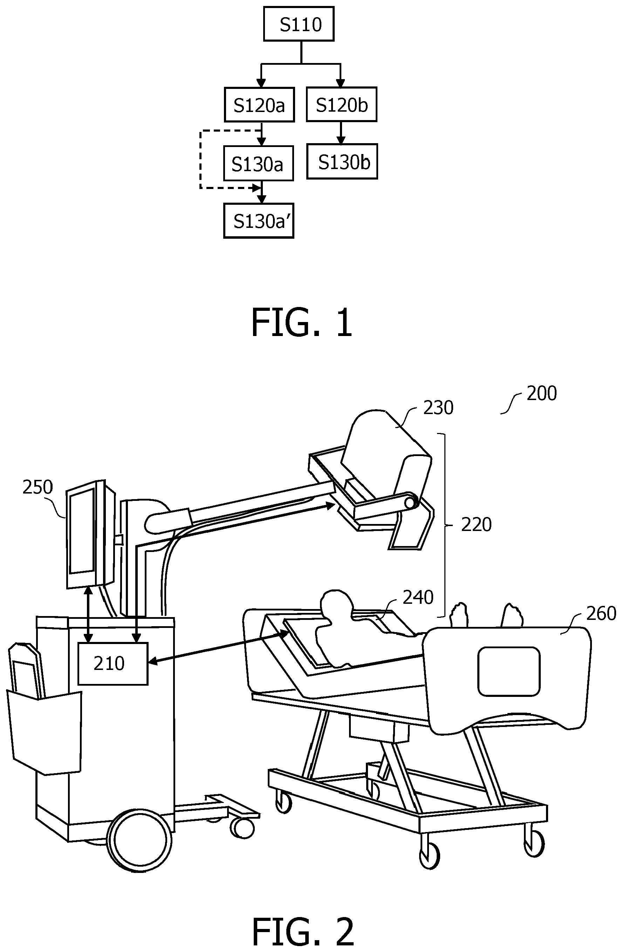

Fig. 1 is a flowchart illustrating an example of a method of compensating for differences in medical images, in accordance with some aspects of the present disclosure. Fig. 2 is a schematic diagram illustrating an example of a system 200 for compensating for differences in medical images, in accordance with some aspects of the present disclosure. Operations described in relation to the method illustrated in Fig. 1, may also be performed in the system 200 illustrated in Fig. 2, and vice versa. With reference to Fig. 1, the computer-implemented method of compensating for differences in medical images, includes:
receiving S110 image data comprising a temporal series of medical images 110_{1..i}, the temporal series including one or more medical images generated by a first type of imaging modality 120, and one or more medical images generated by a second type of imaging modality 130, 130', the first type of imaging modality being different to the second type of imaging modality;
generating S120a, S120b, from the temporal series of medical images 110_{1..i}, a normalised temporal series of medical images 140_{1..i}, or one or more normalised measurement values 150_{1..j} representing a region of interest in the temporal series of medical images 110_{1..i}; and
outputting S130a, S130a', S130b the normalised temporal series of medical images 140_{1..i} and/or one or more measurement values derived from the normalised temporal series of medical images 140_{1..i}, or outputting the one or more normalised measurement values 150_{1..j}, respectively.

In the above method, the normalised temporal series of medical images, the one or more measurement values derived from the normalised temporal series of medical images, and the one or more normalised measurement values, each provide compensation for differences in the type of imaging modality that is used to acquire the images in the temporal series. By providing such compensation, a more reliable comparison between the images can be made, which permits a more reliable assessment to be made of longitudinal changes in a medical condition in the subject.

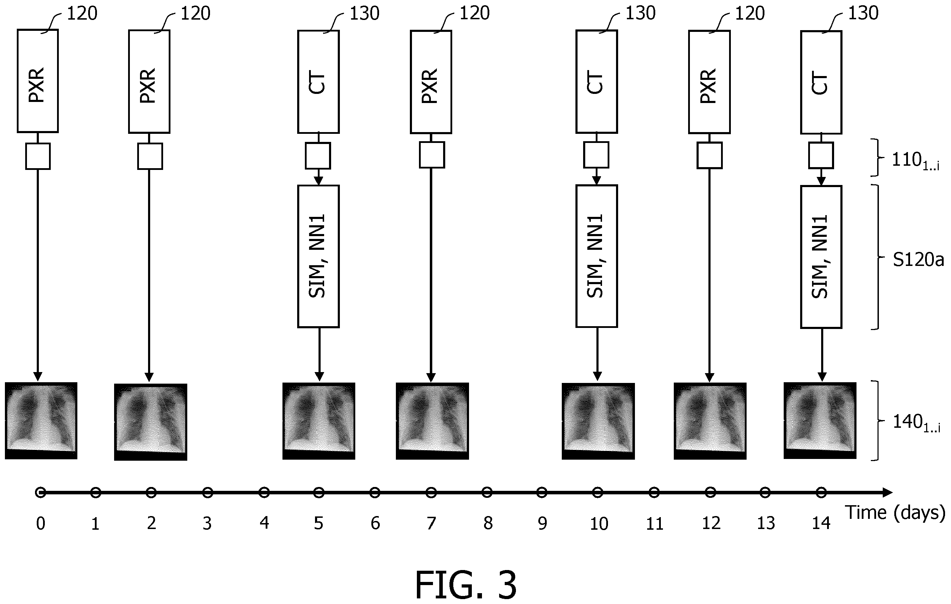

Fig. 3 is a schematic diagram illustrating a first example of a method of compensating for differences in medical images, in accordance with some aspects of the present disclosure. With reference to Fig. 1, and Fig. 3, in the operation S110, image data is received. The image data comprises a temporal series of medical images 110_{1..i}. The received medical images 110_{1..i} form a temporal series in the sense that the images are acquired at different points in time. The images may be acquired periodically, i.e. at regular intervals, or intermittently, i.e. at irregular intervals. The temporal series of medical images may be acquired over a period of time, such as over a period of minutes, hours, days, weeks, or a longer period. The temporal series of medical images 1 10_{1..i} may represent a so-called longitudinal study on a subject. In general, the medical images 110_{1..i} may represent any anatomical region. For instance, the medical images region of interest may be a lung, the heart, the liver, the kidney, and so forth. By way of an example, the temporal series of medical images 1 10_{1..i} that are received in the operation S 110 may represent the chest of the subject, as illustrated in Fig. 3. The temporal series of medical images may for instance represent daily images of the subject's chest that are acquired as part a longitudinal study in a subject to assess the progression of Covid 19 in the lungs of the subject.

The temporal series of medical images 110_{1..i} that is received in the operation S110 include one or more medical images generated by a first type of imaging modality 120, and one or more medical images generated by a second type of imaging modality 130, 130'. The first type of imaging modality is different to the second type of imaging modality. In general, the first imaging modality and the second imaging modality may be any type of imaging modality. By way of some examples, the first type of imaging modality 120 and the second type of imaging modality 130, 130' may be selected from the group: computed tomography, magnetic resonance, ultrasound, and projection X-ray. Computed tomography, magnetic resonance, and ultrasound imaging modalities may be referred to as volumetric imaging modalities in view of their ability to acquire image data for reconstructing volumetric images, i.e. 3D images. A projection X-ray "PXR" imaging modality may be referred-to as a projection imaging modality in view of its ability to acquire image data for reconstructing projection images, i.e. 2D images. Examples of current projection X-ray imaging systems that may generate the 2D medical images 110_{1..i} include the MobileDiagnost M50, which is a mobile X-ray imaging system, the DigitalDiagnost C90, which is ceiling-mounted X-ray imaging system, and the Azurion 7, which includes a source-detector arrangement mounted to a C-arm, all of which are marketed by Philips Healthcare, Best, the Netherlands.

By way of an example, the medical images 1 10_{1..i} that are received in the operation S 110 may include projection X-ray images that are generated by a projection X-ray imaging system labelled "PXR" in Fig. 3, and volumetric images that are generated by a CT imaging system labelled "CT" in Fig. 3. The projection X-ray images illustrated in Fig. 3 may for example be generated by the projection X-ray imaging system 220 illustrated in Fig. 2, which is a so-called mobile X-ray imaging system that is often used in intensive care settings in order to obtain images of subjects with restricted mobility. The projection X-ray imaging system 220 illustrated in Fig. 2 includes an X-ray source 230 and a wirelesslycoupled X-ray detector 240. The versatility of this type of X-ray imaging system facilitates the imaging of subjects in complex settings in which a subject has restricted mobility, such as the bed-bound setting illustrated in Fig. 2. As illustrated in the example in Fig. 3, some of the medical images 110_{1..i} in the temporal series are generated using a projection X-ray imaging system PXR. These images are generated daily, or every few days in the illustrated example. However, as illustrated in the example in Fig. 3, some of the medical images in the temporal series 110_{1..i} are generated by a CT imaging modality, i.e. a different type of imaging modality. The CT imaging modality may have been used to perform additional clinical investigations on a subject, or it may have been used instead of a projection X-ray imaging system in view of imaging system availability. As outlined above, it can be challenging to assess a subject's condition from images that are acquired from different imaging modalities because the CT images are very different in appearance to the X-ray projection images, and there is also no direct correspondence between their image intensities and the image intensities in the X-ray projection images.

Returning to the method illustrated in Fig. 1, in general, the temporal series of medical images 110_{1..i} that is received in the operation S110 may be received via any form of data communication, including for example wired, optical, and wireless communication. By way of some examples, when wired or optical communication is used, the communication may take place via signals transmitted on an electrical or optical cable, and when wireless communication is used, the communication may for example be via RF or optical signals. The temporal series of medical images 110_{1..i} may be received by one or more processors, such as the one or more processors 210 illustrated in Fig. 2, for example. The one or more processors 210 may receive the medical images 110_{1..i} from a medical imaging system, such as the projection X-ray imaging system 220 illustrated in Fig. 2, or from another medical imaging system, or from another source, such as a computer readable storage medium, the Internet, or the Cloud, for example.

With continued reference to Fig. 1, in the operation S120a, a normalised temporal series of medical images 140_{1..i}, is generated from the temporal series of medical images 110_{1..i}. The subsequent operations S130a and S130a' illustrated in Fig. 1 may be performed as alternatives, or in combination. In the operation S130a the normalised temporal series of medical images 140_{1..i} is outputted.

The operation of outputting S 130a the normalised temporal series of medical images 140_{1..i} may be performed in various ways, including displaying, printing, and storing the normalised temporal series of medical images 140_{1..i}. The outputted images may be displayed on a display. For example, the outputted images may be displayed on a display of a tablet, a laptop, or a mobile telephone. Alternatively, the outputted images may be displayed on a monitor, such as the monitor 250 illustrated in Fig. 2, for example. By way of some examples, the normalised temporal series of medical images 140_{1..i} may be displayed side-by side, or as a temporal sequence, or as an overlay image. In another example, the normalised temporal series of medical images 140_{1..i} may be displayed by outputting the images from the first type of imaging modality together with the normalised images generated from the images generated by second type of imaging modality, and overlaying onto the normalised images generated from the images generated by the second type of imaging modality, the images generated by the second imaging modality. The images generated by the second imaging modality are also registered to the normalised images generated from the images generated by the second type of imaging modality. This enables clinical findings from the second type of imaging modality, and which might not be evident from the images from the first type of imaging modality, to be provided to a user in order to assist the user in analysing a medical condition in the subject.

In the operation S 130a', one or more measurement values derived from the normalised temporal series of medical images 140_{1..i}, are outputted. Thus, rather than outputting the normalised temporal series of medical images 140_{1..i}, or in addition to outputting these images, one or more measurement values may be derived from the normalised temporal series of medical images 140_{1..i} and outputted. In general, a measurement value may be outputted for one or more images in the temporal series. For example, a single measurement value may be derived from an individual image in the temporal series, and outputted, or a single measurement value may be derived from multiple images in the temporal series and outputted. In the latter case, a single measurement value may represent a change over time between multiple images. Alternatively, measurement values for each of multiple images ma be outputted.

Various measurement values may be derived from the images and outputted in this respect. In general, the measurement values may represent image intensities, or a diagnostic metric. The measurement values may for example represent an intensity, a volume, or a shape, of a region of interest in the normalised temporal series of medical images 140'_{1..i}. An example of a diagnostic metric is a lung perfusion index. Examples of lung perfusion indices include: a volume of a pneumothorax, i.e. a volume of a "collapsed lung", or an amount of pleural effusion, i.e. an amount of "fluid around the lung", or an amount of pulmonary edema, i.e. an "amount of fluid in the lung", or a stage of pneumonia. Continuing with the example of the lung, the measurement values of the lung perfusion indices may be determined by e.g. contouring the lung in the normalised temporal series of medical images 140'_{1..i}, assigning pixels within the lung to air or to water based on their intensity values, and estimating a volume of the respective air and water regions for each of the normalised temporal series of medical images 140_{1..i}. The value of the lung perfusion index may then be outputted for each of multiple images in the temporal series. Alternatively, a single measurement value may be derived from multiple images in the temporal series and outputted by for example determining individual measurement values for multiple images in the temporal series, and representing the individual measurement values as a rate of change of the lung perfusion index. The measurement value(s) that are derived from the normalised temporal series of medical images 140_{1..i} may also be determined in other ways. In one example, the measurement value(s) that are derived from the normalised temporal series of medical images 140_{1..i} are determined by applying a statistical analysis to the region of interest. In this example, the method described with reference to Fig. 1 may also include:
performing a statistical analysis of image intensity values within a region of interest in the normalised medical images 140_{1..i}; and
wherein the outputting S130a' one or more measurement values derived from the normalised temporal series of medical images 140_{1..i}, comprises:
   outputting the result of the statistical analysis.

Various statistical analysis methods may be applied to the image intensity values within the region of interest in the normalised medical images 140_{1..i}. One example implementation of such a statistical analysis is based on a local patch-based analysis of the pixel-values in the normalised medical images 140_{1..i}. In this example, statistics may be generated for a patch in each of the normalised medical images 140_{1..i}. This may include determining the mean and standard deviation of the pixel values within the patch, assuming e.g. a Gaussian distribution. When comparing corresponding patches of e.g. two images A and B in the normalised medical images 140_{1..i}, the Gaussian statistics of the patch of image A may be used to estimate how likely the measured intensity values in the patch from image B is. This likelihood may be thresholded in order to provide a prediction for a change within the patch. The likelihood may also be visualized as an overlay of images A and B in order permit a reviewing clinician to analyse the detected changes in the images over time. This visualization may consequently permit the reviewing clinician to assess whether the detected changes are statistically relevant, or e.g. whether they only stem from an imperfect normalization procedure.

In a related example, the operation of outputting the result of the statistical analysis for the normalised medical images 140_{1..i} may include displaying a spatial map of the statistical analysis as an overlay on one or more of the normalised medical images 140_{1..i}. Providing the result of the statistical analysis in this manner may facilitate the clinician to provide an efficient analysis of the progression of a medical condition in the subject.

By way of some other examples, the outputting one or more measurement values derived from the normalised temporal series of medical images 140_{1..i} may include outputting a numerical value of the magnitude, e.g. as an absolute value, or a percentage, or outputting the measurement value(s) graphically. In one example, the measurement values may be represented graphically as an overlay on one or more of the normalised medical images 140_{1..i} in the temporal series by representing an increase in lung volume between consecutive images in the series in green, and a decrease in lung volume between consecutive images in the series in red.

By outputting the normalised temporal series of medical images 140_{1..i} and/or one or more measurement values derived from the normalised temporal series of medical images 140_{1..i}, the method illustrated in Fig. 1 facilitates a reviewing clinician to make a more reliable comparison between the region of interest in the images. This, in turn, permits the clinician to make a more accurate assessment of longitudinal changes in the subject's condition.

In general, the normalised temporal series of medical images 140_{1..i} may represent images generated by a common type of imaging modality. In a first set of examples, the common type of imaging modality is a projection imaging modality, and one or more of the images in the normalised temporal series of medical images 140_{1..i} is provided by projecting volumetric medical images. In a second set of examples, the common type of imaging modality is a projection imaging modality, and one or more of the images in the normalised temporal series of medical images 140_{1..i} is provided by inputting medical images into a neural network NN1. The common type of imaging modality may in general be the first type of imaging modality, or the second type of imaging modality, or a different type of imaging modality to the first and second types of imaging modalities.

With reference to the method described above with reference to Fig. 1, in the first set of examples, the generating operation comprises generating S120a a normalised temporal series of medical images 140_{1..i} from the temporal series of medical images 110_{i..i}. The outputting operation comprises outputting S130a, S130a' the normalised temporal series of medical images 140_{1..i} and/or one or more measurement values derived from the normalised temporal series of medical images 140_{1..i}. Moreover, the normalised temporal series of medical images 140_{1..i} represent images generated by a common type of imaging modality.

Thus, in the first set of examples, the normalised temporal series of medical images 140_{1..i} may be outputted, as illustrated in Fig. 3.

Continuing with the first set of examples, in one example, the first type of imaging modality 120 is a projection imaging modality and the second type of imaging modality 130, 130' is a volumetric imaging modality; and the common type of imaging modality is the projection imaging modality. In this example, the normalised temporal series of medical images 140_{1..i} is generated by providing the normalised temporal series of medical images as a combination of the one or more medical images from the temporal series 110_{1..i} that are generated by the first type of imaging modality 120 and one or more projection images, and wherein the one or more projection images are provided by projecting the one or more medical images generated by the second type of imaging modality 130, 130' such that the one or more projected images correspond to one or more of the medical images generated by the first type of imaging modality 120. Thus, the normalised temporal series of medical images 140_{1..i} includes the projection images that are generated by the first type of imaging modality, and the projected images that are generated by projecting the volumetric images generated by the second type of imaging modality.

In this example, the normalised temporal series of medical images 140_{1..i} are therefore projection images, and the volumetric images generated by the second type of imaging modality 130, 130' are projected in order to provide simulated, or "virtual" projection images that correspond to one or more of the projection images generated by the first imaging modality. This example is illustrated in Fig. 3, and wherein some of the images in the normalised temporal series of medical images 140_{1..i} in the lower portion of the figure are provided by the projection images that are generated by the projection X-ray imaging system labelled "PXR", and other "virtual" projection images in the normalised temporal series are provided by projecting the volumetric images that are generated by the CT imaging system labelled "CT".

In general, in the normalised temporal series of medical images 140_{1..i}, the volumetric images may be replaced by, or augmented with, projection images that are generated from the volumetric images at corresponding points in time. The operation of generating the simulated, or "virtual" projection images from the volumetric images is illustrated in Fig. 3 by the operation SIM. In so doing, the outputted normalised temporal series of medical images 140_{1..i}, or the outputted one or more measurement values derived from the normalised temporal series of medical images 140_{1..i}, may be used to identify temporal changes in a region of interest, and consequently to assess the progression of a medical condition in the subject.

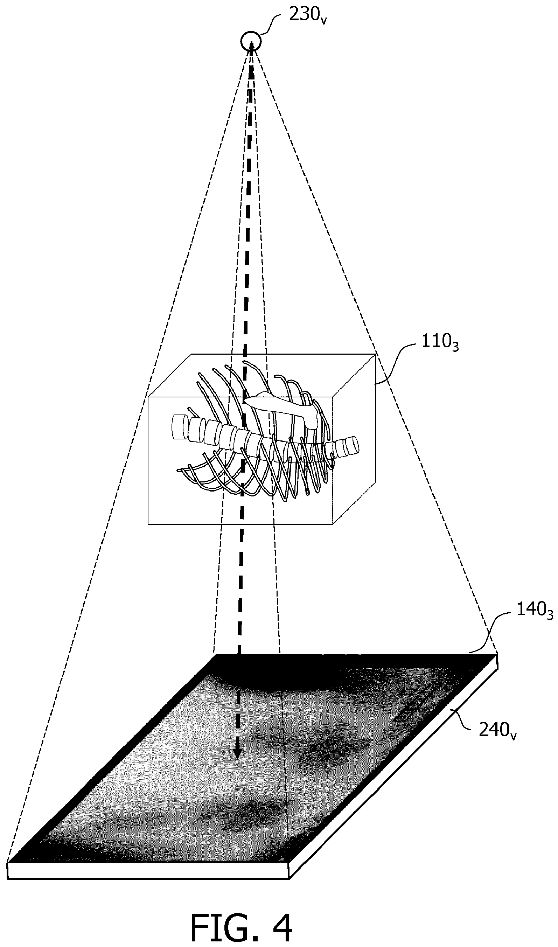

In one example, the operation of projecting the one or more medical images generated by the second type of imaging modality 130, 130' such that the one or more projected images correspond to one or more of the medical images generated by the first type of imaging modality 120, comprises projecting the one or more medical images generated by the second type of imaging modality onto a virtual detector 240ᵥ using a virtual source 230ᵥ. This example is described with reference to Fig. 3 and Fig. 4. In another example, described later, a neural network NN1 is used to generate projection images.

With reference to Fig. 3, in this example, the first imaging modality 120 is a projection modality. The first imaging modality 120 may for example be the projection X-ray modality labelled "PXR" in Fig. 3, and which generates projection X-ray images in the temporal series 110_{1..i} at times corresponding to day 0, day 2, day7, and day 12. In this example, the second imaging modality is a volumetric imaging modality. The second imaging modality may for example be the CT imaging modality labelled "CT" in Fig. 3, and which generates volumetric CT images at times corresponding to day 5, day 10, and day 14. Fig. 4 is a schematic diagram illustrating an example of the operation of projecting a volumetric medical image 110₃ onto a virtual detector 240ᵥ using a virtual source 230ᵥ, in accordance with some aspects of the present disclosure. With reference to Fig. 4, the example volumetric image generated at day 3, i.e. the CT image 110₃ representing the chest, is projected onto a virtual detector 240ᵥ in order to generate the normalised medical image 140₃. The normalised medical image 140₃ is a projection image that corresponds to the projection X-ray images generated by the first imaging modality 120, i.e. a projection X-ray imaging modality. The normalized medical image 140₃ is then included in the normalised temporal series of medical images 140_{1..i} at a time corresponding to the time when the volumetric image was generated, i.e. on day 5.

The operation of projecting the volumetric image 110₃ onto the virtual detector may be performed mathematically using a ray-tracing operation. With reference to Fig. 4, in this operation, the density values represented by the volumetric image 110₃ are integrated along the paths of virtual rays, such as the thick dashed line, that are projected from the virtual X-ray source 230ᵥ, through the volumetric image 110₃, and onto the virtual X-ray detector 240ᵥ, in order to provide integrated density values at positions on the virtual X-ray detector 240ᵥ. The integrated density values may also be adjusted to compensate for X-ray scatter. The projection image 140₃ is generated by performing this ray-tracing operation and calculating the integrated density values at multiple positions across the virtual X-ray detector 240ᵥ. These operations are performed mathematically, i.e. using a virtual source 230ᵥ and a virtual detector 240ᵥ.

The operation of projecting the volumetric image 110₃ onto the virtual detector described above may be performed based on a known relative positioning between the virtual source, the virtual detector, and a subject represented in the one or more medical images generated by the second type of imaging modality 120. This provides that the projected image(s) correspond to one or more of the medical images generated by the first type of imaging modality 120. The relative positioning of the virtual source and the virtual detector may be known from the relative position of the actual source and the actual detector of the first imaging modality with respect to the subject at the time of generating the projection images with the first type of imaging modality. These relative positions may be known by performing the imaging using a standard clinical imaging protocol. The protocol may define parameters such as the separation between the actual X-ray source and the actual detector, the position of the subject with respect to the detector, the image resolution, and so forth. For instance, if a so-called "PA" projection image is generated by the first imaging modality with the subject erect and facing an upright X-ray detector and with specified distances between the subject and each of the X-ray source and X-ray detector, then the same relative positions would be used to generate the projection images using the virtual source, the virtual detector, and the subject in the volumetric image(s) generated by the second type of imaging modality. Alternatively, the relative positions may be measured at the time of generating the projection images with the first imaging modality. For example, the relative positions may be measured using a (depth) camera that captures the actual X-ray source, the actual X-ray detector, and the subject, at the time of generating the projection images.

Alternatively or additionally, the operation of projecting the volumetric image 110₃ onto the virtual detector may include adjusting a relative positioning between the virtual source, the virtual detector, and the one or more medical images generated by the second type of imaging modality 130, 130' such that a shape of one or more anatomical features in the projected one or more images corresponds to a shape of the one or more corresponding anatomical features in the one or more medical images generated by the first type of imaging modality 120. In this case, the shape correspondence provides that the projected image(s) correspond to one or more of the medical images generated by the first type of imaging modality 120. In this case, an optimisation process is executed wherein the relative positioning between the virtual source, the virtual detector, and the one or more medical images generated by the second type of imaging modality 130, 130', are adjusted iteratively until a difference between the shape of one or more anatomical features in the projected image(s), and the shape of the one or more corresponding anatomical features in the one or more medical images generated by the first type of imaging modality 120, is less than a predetermined threshold value. A starting point for the optimisation may be the rough relative positions that is expected for the type of image and the region of interest in the image. An alternative starting point for the optimisation may be the expected relative positions from the standard imaging protocol, or the measured relative positions that are measured using the (depth) camera. In this example, the shape of the anatomical features in the images may be determined by applying known image segmentation algorithms to the respective images.

In these examples in which the volumetric image is projected onto a virtual detector, the operation of projecting the one or more medical images generated by the second type of imaging modality onto a virtual detector 240ᵥ using a virtual source 230ᵥ may be performed based additionally on known operational parameters of the projection imaging modality, i.e. the first type of imaging modality. For example, if the first imaging modality is a projection X-ray imaging modality, the projecting operation may be performed based additionally on one or more operational parameters such as the kVₚ energy of the X-ray source, the X-ray dose, the exposure time, the sensitivity of the X-ray detector, and so forth. By additionally using such parameters, improved correspondence may be achieved between the projected image 140₃, and the one or more medical images generated by the first type of imaging modality 120.

As mentioned above, in addition to the challenge presented by images in a temporal series being acquired using different types of imaging modalities during the course of a clinical investigation, there may also be differences in the manner in which the medical images in the temporal sequence 110_{1..i} illustrated in Fig. 3, are acquired. For example, there may be differences in the subject's posture, or differences in the viewing angle of a medical imaging system, or differences in the amount of ionising radiation dose used to acquire the images. Such factors may be present even when images are acquired from a single type of imaging modality. These factors exacerbate the challenge of identifying temporal changes in the region of interest, and can even lead to an erroneous diagnosis of a subject's condition.

One or more further operations may be performed on the normalised temporal sequence of medical images 140_{1..i} in order to compensate for the effects of these differences, and to thereby further improve the identification of temporal changes in a region of interest, and to consequently improve the assessment of the progression of a medical condition in the subject.

With reference to the method illustrated in Fig. 1, in one example, the operation of generating S120a a normalised temporal series of medical images 140_{i..i} includes warping one or more of the images in the normalised temporal series of medical images 140_{1..i} such that a shape of one or more anatomical features 160 in the warped one or more images 140'_{1, 2} corresponds to a shape of the one or more anatomical features 160 in a reference image 170.

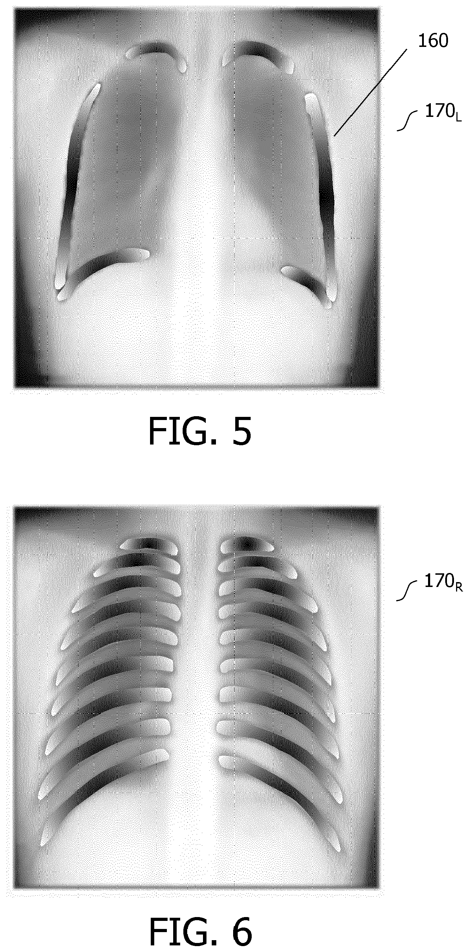

In this example, the effect of the warping is to provide images in which the anatomical features have a similar shape. This enables a more accurate comparison to be made between the images in the normalised temporal series of medical images 140_{1..i}. In this example, the reference image 170 may be provided by an image from the received temporal series of medical images 110_{1..i}, or an image from the normalised temporal series of medical images 140_{1..i}, or an atlas image. By way of some examples, Fig. 5 illustrates an example of an atlas image 170_{L} for the lung field, in accordance with some aspects of the present disclosure, and Fig. 6 illustrates an example of an atlas image 170_{R} for the rib cage, in accordance with some aspects of the present disclosure. The warping operation in this example may for example include warping one or more of the images in the normalised temporal series of medical images 140_{1..i} such that a shape of the lungs, or the shape of the rib cage, in the warped one or more images corresponds to a shape of the lungs, or the shape of the rib cage in the atlas images illustrated in Fig. 5 and Fig. 6.

The atlas images 170_{L,R} illustrated in Fig. 5 and Fig. 6 indicate by way of their intensity values, a probability of a structure belonging to a lung border, and to the ribs, respectively. The atlas image 170_{L,R} may be received by the one or more processors 210 illustrated in Fig. 2 for example. The atlas image may be received from a database of atlas images. The atlas image may be selected from the database based on similarities between the subject that is imaged in the temporal series of medical images 110_{1..i}, and a reference subject represented in the atlas image 170_{L,R}. For example, the atlas image may be selected based on similarities between age, gender, and size of the subject and the reference subject. The atlas image 170_{L,R} provides a reference shape for the anatomical features 160, i.e. for the lungs, or for the ribs. The atlas image may represent a preferred perspective of the anatomical features in the reference subject. The atlas image may be acquired whilst the reference subject maintains a preferred posture. By way of an example, if the anatomical feature is the lungs, an atlas image for the lungs might be a so-called posteroanterior "PA" chest view that represents the lungs, bony thoracic cavity, mediastinum and great vessels. Such an atlas image may be acquired with the subject erect and facing an upright X-ray detector, at a specified state of inspiration, with the superior aspect of the X-ray detector a specified distance above the shoulder joints, with the chin is raised as to be out of the image field, with the shoulders rotated anteriorly to allow the scapulae to move laterally off the lung fields, and under specified operating settings (e.g. X-ray kVp energy, and exposure time) of the X-ray imaging system.

In the warping operation, the images in the normalised temporal series of medical images 140_{1..i} are warped to the atlas image 170_{L,R}. The warping operation may be performed using various known transforms. One example of a suitable transformation is an affine transform. Other examples of suitable transformations include B-splines, thin-plate splines, and radial basis functions. The warping operation may be performed based on a mapping between a plurality of corresponding landmarks 180_{i..j} represented in both the warped image and the reference image 170. The warping operation may be performed using a neural network. If the warping is performed by a neural network, the neural network may or may not explicitly use such landmarks. The landmarks 180_{1..j} may be provided by anatomical features, or by fiducial markers. By way of some examples, anatomical features such as bones, e.g. ribs, the scapula, and so forth, or organ contours, e.g. a contour of the lung, the diaphragm, the heart shadow, and so forth, may serve as anatomical landmarks. Such features are identifiable in the normalised temporal series of medical images 140_{i..i} by virtue of their X-ray attenuation. Fiducial markers that are formed from X-ray attenuating materials are also identifiable in the medical images and these may also serve as landmarks. The fiducial landmarks may be located superficially, or within the body, and at known reference positions. In the latter case, the fiducial markers may have been implanted for use as a surgical guide for example. An implanted device such as a pacemaker may also serve as a fiducial marker. Fiducial markers may also be provided by interventional devices that are inserted within the body.

The landmarks 180_{i..j} in the normalised temporal series of medical images 140_{i..i}, and the corresponding landmarks in the reference image 170, may be identified using various techniques. In one example, a feature detector is used to identify anatomical landmarks in the normalised temporal series of medical images 140_{i..i}. The identified landmarks are then mapped to corresponding anatomical landmarks that are labelled in the reference image 170. In this example, the reference image 170 includes a plurality of labelled anatomical landmarks; and the method described with reference to Fig. 1 also includes:
applying a feature detector to each medical image in the normalised temporal series of medical images 140_{i..i} to identify a plurality of landmarks in the medical image that correspond to the labelled anatomical landmarks; and
wherein the mapping is determined using the identified corresponding landmarks.

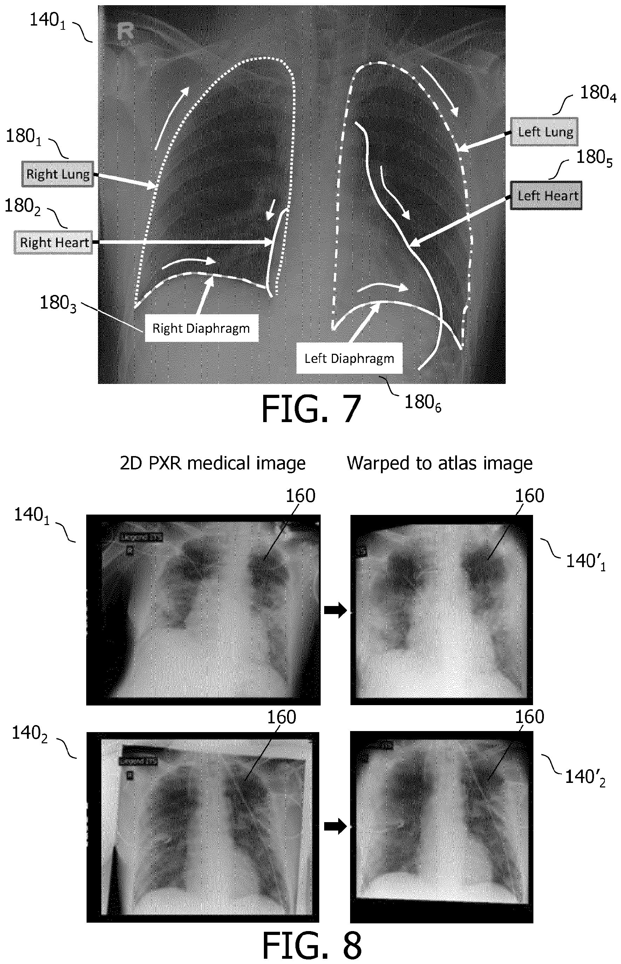

In this example, the operation of applying a feature detector to the medical images in the temporal series, may be performed using an edge detector, or a model-based segmentation, or a neural network, for example. By way of an example, Fig. 7 illustrates an example of the result of applying feature detector to a medical image 140₁ to identify a plurality of landmarks in the medical image, in accordance with some aspects of the present disclosure. The medical image 140₁ illustrated in Fig. 7 represents the chest, and includes bone regions such as the ribs and the spine, and an outline of the heart shadow and the lungs. In this example, the anatomical region that is used in the warping operation is the lungs, and the feature detector has identified in the medical image 140₁, outlines representing the right lung, the right side of the heart shadow, the right side of the diaphragm, the left lung, the left side of the heart shadow, and the left side of the diaphragm as landmarks 180_{1..6} respectively. These landmarks correspond to landmarks in an atlas image, such as the atlas image 170_{L} for the lung field illustrated in Fig. 5. The landmarks 180_{1..6} that have been identified in the medical image 140₁, are then mapped to their corresponding landmarks in the atlas image 170_{L}, in order to warp the medical image 140₁ to the atlas image in the warping operation.

The result of the warping operation is to provide warped medical images 140'_{1..i}, in which a shape of one or more anatomical features 160 corresponds to a shape of the one or more anatomical features 160 in the reference image 170. The correspondence may be measured by comparing a shape of the anatomical feature(s) in the images using e.g. a measurement of the distance between corresponding landmarks in the warped medical images 140'_{1..i} and the reference image 170. Alternatively, the correspondence may be measured by calculating a value of the Dice coefficient between the segmentation mask in the warped medical images 140'_{1..i} and the segmentation mask in the reference image 170. When the calculated value of such measures is within a predetermined range, the shapes may be deemed to correspond to one another. The effect of the warping operation is illustrated in Fig. 8, which illustrates an example of the warping of two 2D medical images in a temporal series, 140₁, 140₂, to a reference image, in accordance with some aspects of the present disclosure. The two 2D medical images 140₁, 140₂, illustrated on the left-hand side of Fig. 8 are projection X-ray images that are generated by the same type of imaging modality, a projection X-ray "PXR" imaging system. Although the images 140₁, and 140₂ represent the same anatomical region in a subject, i.e. the lungs, the images are acquired with the subject in a different pose. The differences in the subject's pose confound the identification of temporal changes in a medical condition in the lungs between the images 140₁, and 140₂. The warped images 140'₁ and 140'₂, on the right-hand side of Fig. 8 have been warped such that the lungs of the subject corresponds to the lung field in the atlas image, as described above. As illustrated in the warped images 140'₁ and 140'₂, the warping operation has the effect of compensating for the differences in the subject's pose when the images 140₁, and 140₂ were acquired. Thus, the warping operation permits a more reliable identification of temporal changes in the subject's lungs, and consequently a more reliable assessment to be made of the progression of a medical condition in the subject

As mentioned above, other operations may also be performed on the normalised temporal series of medical images 140_{i..i} in order to compensate for differences in the manner in which the medical images are acquired. These operations may be performed instead of the warping operation, or in addition to the warping operation. The inventors have observed that not only can the assessment of temporal changes in medical images be hampered by differences in the shape of an anatomical region, between the images, but that the assessment of such changes may also be hampered by differences in the intensity scale of the images. More specifically, it is differences in the intensity scale that arise from differences in the way in which medical images in a temporal series are acquired, that can hamper their comparison. By way of an example, the intensity at any point in a 2D X-ray image is dependent on the values of the X-ray energy "kVp", the exposure time, and the sensitivity of the X-ray detector that are used to acquire the image. With reference to Fig. 3, since the images in a temporal series 110_{1..i} are acquired over a period of time, the images in the temporal series may be generated by projection X-ray imaging systems "PXR" that have different settings, or indeed they may be generated by different X-ray imaging systems. In contrast to computed tomography images, and in which a Hounsfield unit value may be assigned to each image voxel, in 2D projection X-ray images there is no corresponding absolute attenuation scale for the pixel intensity values.

In one example the operation of generating S120a a normalised temporal series of medical images 140_{i..i} comprises:
adjusting an intensity of the images in the normalised temporal series of medical images 140_{1..i} based on an intensity at one or more positions in a reference image 170;
   and/or
adjusting an intensity of the images in the normalised temporal series of medical images 140_{1..i} using an image style transfer algorithm.

These adjusting operations provide compensation is provided for inter-image variability arising from differences in the way in which the medical images are acquired.

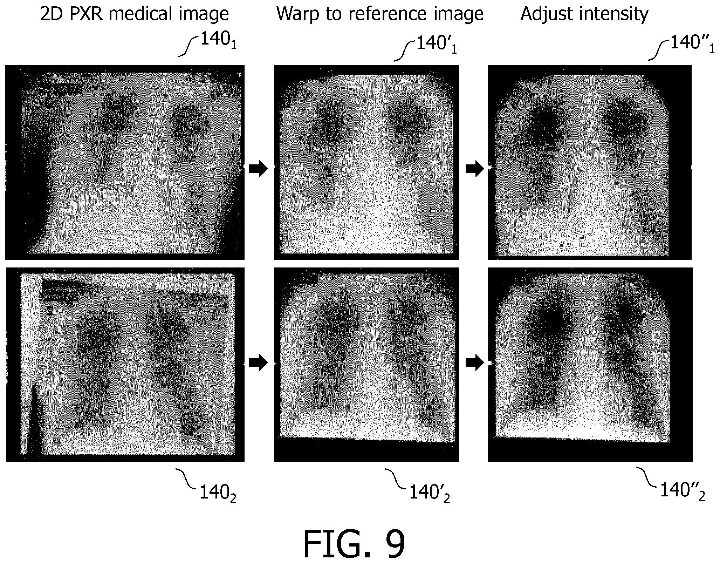

This example is described with reference to Fig. 9, which illustrates an example of the warping of two 2D medical images in a temporal series, 140₁, 140₂, to a reference image, and the adjustment of the intensity of the medical images, in accordance with some aspects of the present disclosure. The warping operation that is performed on the 2D Projection X-ray "PXR" medical images 140₁, and 140₂, to provide the warped images 140'₁, and 140'₂, in this example, is performed as described above.

In this example, positions may be defined in the reference image 170 manually, or automatically. By way of an example, if the normalised temporal series of medical images 140_{1..i} in Fig. 9 represent the lungs, positions in the reference image 170 illustrated in Fig. 5 may be defined within the lung field, or within the mediastinum. The positions may be pre-defined in the reference image 170, or defined based on user input that is provided via a user input device in combination with a displayed image. Techniques such as windowing and histogram normalisation are then used to adjust the intensity values of the normalised temporal series of medical images 140'₁ and 140'₂ in the centre of Fig. 9, in order to provide the intensity-adjusted images 140"₁ and 140"₂ on the right-had side of Fig. 9.

Alternatively, or additionally, an image style transfer algorithm may be used to provide the intensity-adjusted images 140"₁ and 140"₂. Various image style transfer transforms are known for this purpose. These may be based on e.g. a Laplace pyramid, or a (convolutional) neural network, for example. An example of an image style transfer algorithm is disclosed in the document WO 2013/042018 A1. This document discloses a technique for transforming a slave image that involves generating a color or grey scale transformation based on a master image and a slave image. The transformation is used to optically adapt the slave image to the master image. This technique may be used to adjust an intensity of the medical images 140_{1..i} in the temporal series such that the adjusted intensity values in the normalised temporal series of medical images 140'_{1..i} have a similar appearance, or style. The technique disclosed in this document may be applied by using the medical images 140_{1..i} in the temporal series as the slave images, and using a reference image, such as the atlas image illustrated in Fig. 5 and Fig. 6, or one of the medical images 110_{1..i} in the temporal series, as the master image.

In a related example, the operation of adjusting an intensity of the images in the normalised temporal series of medical images 140_{1..i} based on an intensity at one or more positions in a reference image 170, may include:
calculating an average intensity value within a portion of the reference image 170; and
normalising the intensity values in each medical image in the normalised temporal series of medical images 140_{1..i} using the average intensity value.

In this example, the portion of the reference image 170 that is used to normalise the intensity values in each medical image 140_{1..i} may be selected as a portion of the reference image in which the image intensity is expected to be temporally-invariant over a duration of the temporal series of medical images 110_{1..i}. For instance, if the region of interest is a lung, the selected portion of the reference image may correspond to a portion of the spine since the attenuation of the spine would not be expected to change overtime. Alternatively, if the region of interest is a lung, the selected portion may correspond to a portion of the lung in the reference image that is expected to be free of disease in the temporal series of medical images 110_{1..i}. For instance, if the subject's condition under investigation is pulmonary edema, then a collection of fluid over time may be expected to result in temporal changes in the image intensity in the lower portion of the lung, whereas the upper portion of the lung may be expected to remain free of disease and consequently to have a stable image intensity. In this case, the upper portion of the lung in the reference image 170 may be used as the portion of the reference image 170 that is used to normalise the intensity values in the medical images 140_{1..i}.

The portion of the lung in the reference image 170 may be identified automatically, or based on user input received via a user input device in combination with a display device. The portion of the reference image 170 may represent a combination of air in the lung, and ribs surrounding the lung. An average intensity of the pixel values in this portion of the reference image may then be calculated. This average value may then be used to normalise the intensity values of the medical images 140_{1..i} in the temporal series. With reference to Fig. 5, this may for instance include identifying a disease-free corresponding portion of the lung, e.g. towards the top of the lung in the lung field of the atlas image, calculating an average value of the intensity values in the corresponding disease-free portion, calculating a scaling factor as a ratio of the average intensity values in the disease-free region of the medical image 140₁ to the average intensity values in the portion of the lung in the atlas image, and scaling the image intensity values in the medical images 140_{1..i} using the scaling factor to provide adjusted intensity values in the intensity-adjusted medical images 140"₁, 140"₂. These operations are then be repeated for the other medical images 140_{1..i} in the temporal series. Since the portion of the lung in the atlas image is used to normalise each medical image 140_{1..i} in the temporal series, the medical images are scaled to a common reference intensity, which facilitates a more reliable comparison to be made between the image intensities in other portions of the images.

Additional portions of the reference image 170 may also be used to perform the intensity normalisation described above. For example, a second region of interest may be identified within the mediastinum, or within a bone region, of the reference image, and also used to perform the intensity normalisation. The use of two regions of interest having different attenuation values to normalise the intensity in the medical images 140_{1..i} in the temporal series may facilitate a more accurate mapping of the intensity values, and consequently a more reliable identification of changes in the regions of interest in the images.

In this example, a value of a quality metric may also be calculated and outputted for the adjusted intensity values in the normalised medical images 140"_{1..i}. The value of the quality metric may be calculated based on e.g. the statistics of the image intensity values in the portion of the reference image, or the corresponding portion of the medical image 1 10_{1..i}, or based on the quality of the mapping between the landmarks used in the warping operation. The value of the quality metric may be relatively lower if the variance of the image intensity values in the portion of the reference image is high, and vice versa. Outputting the value of the quality metric re-assures a reviewing clinician of the accuracy of the method.

The inventors have also observed that subsequent to the warping operation described above, some regions in the normalised series of medical images 140'_{1..i} that are outside of the anatomical region that is used for the warping, may be warped to an unnatural shape. This can be confusing to a reviewing physician. In another example, the method described with reference to Fig. 1 may also include:
receiving input defining a region of interest in the received temporal series of medical images 110_{1..i}; and
suppressing one or more image features outside the region of interest, or within the region of interest, in the normalised temporal series of medical images 140_{1..i}.

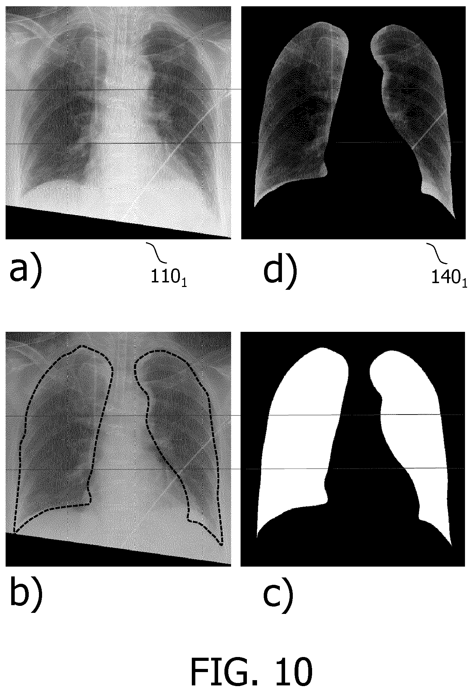

By suppressing the one or more image features in accordance with this example, it is avoided that a reviewing clinician is presented with confounding information. This example is described with reference to Fig. 10, which illustrates an example of the suppression of image features outside a lung region of interest in medical image 140₁, in accordance with some aspects of the present disclosure. Fig. 10a illustrates an image from the received temporal series of medical images 110₁. Fig. 10b illustrates, by way of the dashed lines, a region of interest in the image 110₁. The region of interest may be defined automatically in the image 110₁. For example, a segmentation algorithm, or a feature detector, may be used to automatically define the lung region of interest. Alternatively, the region of interest may be defined manually based on user input received via a user input device in combination with a display device which displays the image 110₁. In Fig. 10c, the outline of the region of interest is illustrated, and this is used to defined a binary mask that is applied to the image 110₁ in order to suppress the features outside the lung region of interest. Fig. 10d illustrates a normalised medical image 140₁ in which the features outside the lung region of interest have been suppressed. As may be appreciated, confounding features outside the lung region of interest are omitted the image illustrated in Fig. 10d.

In the example illustrated in Fig. 10, the image features outside the lung region of interest have been suppressed by mapping their pixel values to a black-level pixel intensity value. However, these features may alternatively be suppressed in a different manner. For example, their pixel values may be mapped to a different pixel intensity value than a black-level pixel intensity value, or their pixel values may be adjusted gradually to a predetermined value in order to "feather-out" the region of interest. The operation of suppressing the image features outside the region of interest may also be performed using a reference image, such as the alas image. For instance, in this example, the method described with reference to Fig. 1 may also include:
receiving input identifying the region of interest in the reference image 170;
mapping an outline of the region of interest from the reference image 170 to each of the normalised medical images 140_{1..i} and
adjusting image intensity values in the normalised medical images 140_{1..i} outside the mapped outline to suppress the one or more image features outside the region of interest.

In this example, various example regions of interest may be pre-defined in the reference image and used to suppress the one or more image features outside the region of interest. Alternatively, the regions of interest may be defined by segmenting the reference image. Since in this example the outline from the reference image is used to suppress the one or more image features outside the region of interest, a reliable outline is used.

Another operation that may be performed on the normalised temporal series of medical images 140_{i..i} in order to compensate for differences in the manner in which the medical images are acquired, is the removal of foreign objects in the images. Foreign objects such as implanted devices may be identified in the images based on their X-ray attenuation values. A feature detector, or a neural network, may be used for this purpose. The image intensity values of the foreign objects set to a default level such as a black level, or a white level, in order to avoid that their presence distracts a reviewing clinician.

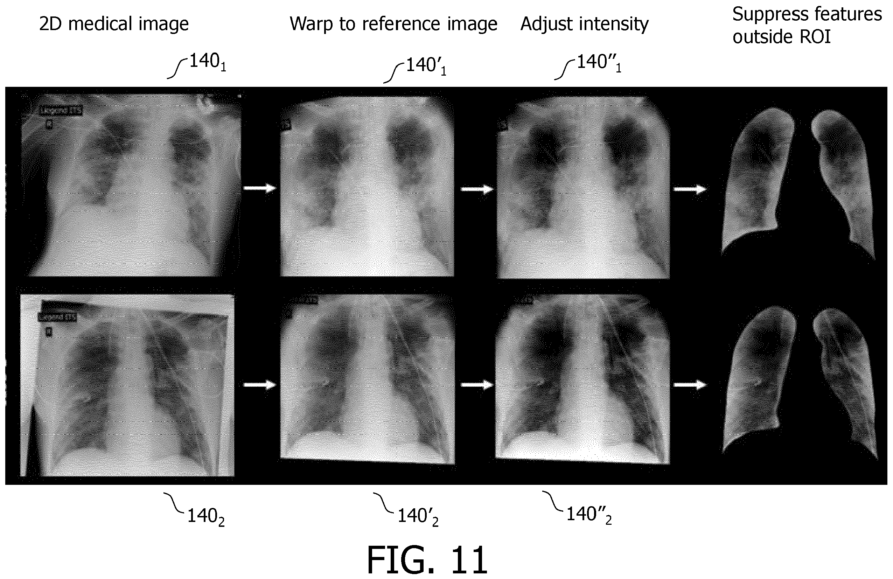

By way of a further example, Fig. 11 illustrates an example of the warping of two normalised medical images 140₁, 140₂, to a reference image, and the adjusting of image intensity values in the images, and the suppression of image features outside a lung region of interest, in accordance with some aspects of the present disclosure. Fig. 11 illustrates the effect of the combined operations of warping, adjusting image intensity values, and suppression of features outside a region of interest, and is a further illustration of the reduction in the amount of variability arising from differences in the manner in which medical images are acquired during a longitudinal study on a subject, that can be achieved in accordance with the method described herein.

As mentioned above, instead of providing the one or more of the images in the normalised temporal series of medical images 140_{1..i} by projecting volumetric medical images, in a second set of examples, one or more of the images in the normalised temporal series of medical images 140_{1..i} is provided by inputting medical images into a neural network NN1. The second set of examples is described with reference to the method illustrated in Fig. 1 and Fig. 3.

In the second set of examples, the first type of imaging modality 120 is a projection imaging modality and the second type of imaging modality 130, 130' is a volumetric imaging modality. The common type of imaging modality is the projection imaging modality. The normalised temporal series of medical images 140_{1..i} is generated by providing the normalised temporal series of medical images as a combination of the one or more medical images from the temporal series 110_{1..i} that are generated by the first type of imaging modality 120 and one or more projection images. The one or more projection images are provided by inputting the one or more medical images generated by the second type of imaging modality 130, 130' into a neural network NN1. The neural network NN1 is trained to generate a projection image corresponding to the first type of imaging modality 120 for each of the inputted images.

The neural network NN1 may be provided by a variety of architectures, including for example a convolutional neural network CNN, a Cycle GAN, or a transformer architecture, for example. In general, the training of a neural network involves inputting a training dataset into the neural network, and iteratively adjusting the neural network's parameters until the trained neural network provides an accurate output. Training is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

The process of training the neural network NN1 illustrated in Fig. 3 therefore includes adjusting its parameters. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean squared error, or the Huber loss, or the cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

In one example, the neural network NN1 is trained to generate a projection image corresponding to the first type of imaging modality 120 for each of the inputted images by:
receiving training data, the training data comprising a plurality of volumetric training images representing the region of interest, the volumetric training images being generated by the second type of imaging modality 130, 130';
receiving ground truth data, the ground truth data comprising, for each of the volumetric training images, a corresponding ground truth projection image generated by the first type of imaging modality 120;
inputting the training data into the neural network NN1; and
for each of a plurality of the inputted volumetric training images:
   predicting, using the neural network NN1, a corresponding projection image;
   adjusting parameters of the neural network NN2 based on a difference between the predicted projection image and the ground truth projection image; and
   repeating the predicting and the adjusting, until a stopping criterion is met.

By way of an example, the first type of imaging modality may be a projection X-ray imaging modality and the second type of imaging modality may be a CT imaging modality. In this example, the training data may include volumetric images of the chest that are generated by the CT imaging modality. The training data may include hundreds, or thousands, or more of such images. The ground truth data includes projection images of the chest region that are generated by the X-ray projection imaging modality. The ground truth data may be provided by pseudo-X-ray projection images that are generated from the volumetric training images of the chest that are generated by the CT imaging modality, and which volumetric images are projected so as to provide pseudo X-ray projection images. The projection may be performed in accordance with the techniques described above. For example, the volumetric images may be projected from a predefined orientation, and using predefined values for a dose of X-ray source, exposure time, and sensitivity of the X-ray detector, in order to provide the ground truth pseudo-X-ray projection images. The ground truth projection images may alternatively be provided by a projection imaging system, in this example, a projection X-ray imaging system. The adjustment of the parameters of the neural network NN1 may be performed in accordance with the backpropagation techniques described above. The resulting trained neural network NN1 is trained such that when provided with a new CT image, it outputs a corresponding projection image. The neural network NN1 may similarly be trained to predict X-ray projection images from images that are generated by different imaging modality to a CT imaging modality. For example, the first imaging modality may alternatively be an MRI imaging modality, and the neural network NN1 trained to predict corresponding X-ray projection images. In this case, the corresponding ground truth data may be provided by a projection X-ray imaging system. The training of the neural network NN1 is then performed in a similar manner.

Having provided the normalised temporal series of medical images 140_{1..i} as a combination of the one or more medical images from the temporal series 110_{1..i} that are generated by the first type of imaging modality 120 and the one or more projection images generated by the neural network NN1; the warping the medical images 140i..i described above, and likewise the adjusting an intensity of the images in the normalised temporal series of medical images 140_{1..i}, and the suppression of one or more image features outside the region of interest, may subsequently be performed on the normalised temporal series of medical images 140_{1..i} in order to further reduce the effects of differences in the manner in which the images were acquired.

In a third set of examples, the right-hand branch of the method illustrated in the flowchart in Fig. 1, is followed. In the third set of examples, one or more normalised measurement values 150_{1..j} representing a region of interest in the temporal series of medical images 110_{1..i} are generated, and the one or more normalised measurement values 150_{1..j}, are outputted. Thus, in contrast to the first and second sets of examples, there is no operation of generating a temporal normalised temporal series of medical images 140_{1..i}, and instead, one or one or more normalised measurement values 150_{1..j} are generated directly from the temporal series of medical images 110_{1..i}.

With reference to the method described above with reference to Fig. 1, the operation of generating comprises generating S120b, from the temporal series of medical images 110_{1..i}, one or more normalised measurement values 150_{1..i} representing a region of interest in the temporal series of medical images 110_{1..i}. The operation of outputting comprises outputting S130b the one or more normalised measurement values 150_{1..i}. The one or more normalised measurement values 150_{1..i} representing the region of interest are generated by:
inputting the temporal series of medical images 110_{1..i} into a neural network NN2; and
wherein the neural network NN2 is trained to generate the normalised measurement value 150_{1..i} representing the region of interest for each of the inputted images 110_{1..i}.

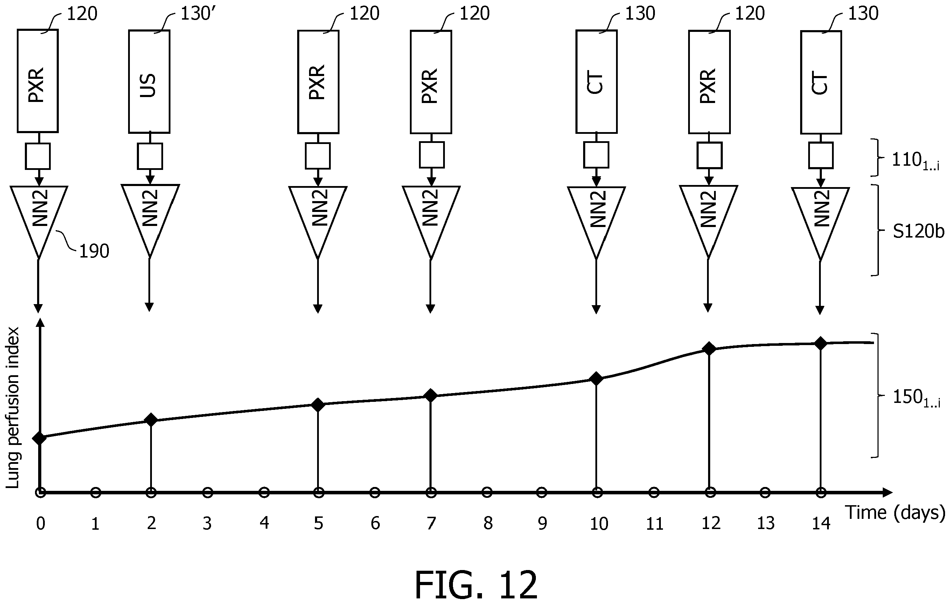

The third set of examples is described with reference to Fig. 12, which is a schematic diagram illustrating a second example of a method of compensating for differences in medical images, in accordance with some aspects of the present disclosure.

With reference to Fig, 12, the first imaging modality 120 may for example be a projection X-ray imaging modality labelled "PXR". The second imaging modality may be a CT imaging modality 130 labelled "CT", or it may be a 3D ultrasound imaging modality 130' labelled "US". In general, the normalised measurement values predicted by the second neural network NN2 may represent image intensities, or a diagnostic metric, such as a lung perfusion index. The measurement values may for example represent an intensity, a volume, or a shape, of a region of interest in the normalised temporal series of medical images 140'_{1..i}. An example of a diagnostic metric is a lung perfusion index. In the illustrated example, the the normalised measurement value 150_{1..i} is a lung perfusion index. In the example illustrated in Fig. 12, the normalised measurement values 150_{1..i}, i.e. the values of the lung perfusion index, are generated by inputting the temporal series of medical images 1 10_{1..i} that are generated by the projection X-ray imaging modality "PXR, or the CT imaging modality "CT", or the 3D ultrasound imaging modality "US" into a neural network NN2.

The second neural network NN2 may be provided by a variety of architectures, including a convolutional neural network CNN, or a transformer architecture, for example. In one example, the neural network NN2 is trained to generate the normalised measurement value 150_{1..i} representing the region of interest for each of the inputted images by:
receiving training data, the training data comprising a plurality of training images representing the region of interest, the training images including a plurality of images generated by the first type of imaging modality 120 and a plurality of images generated by the second type of imaging modality 130, 130';
receiving ground truth data, the ground truth data comprising, for each of the training images, a corresponding ground truth value of the normalised measurement value representing the region of interest;
inputting the training data into the neural network NN2; and
for each of a plurality of the inputted training images:
   predicting, using the neural network NN2, a value of the normalised measurement value representing the region of interest;
   adjusting parameters of the neural network NN2 based on a difference between the predicted value of the normalised measurement value and the corresponding ground truth value; and
   repeating the predicting and the adjusting, until a stopping criterion is met.

The second neural network may be trained to generate the normalised measurement value 150_{1..i} representing the region of interest for each of the inputted images 110_{1..i} using the techniques described above.

By way of an example, the first type of imaging modality may be a projection X-ray imaging modality and the second type of imaging modality may be a CT imaging modality. In this example, the training data may include X-ray projection images of the chest that are generated by the X-ray projection imaging modality, and volumetric images of the chest that are generated by the CT imaging modality. The training data may include hundreds, or thousands, or more of such images. The normalised measurement value that is predicted by the second neural network NN2 may for instance be a lung perfusion index. The ground truth data may be provided by expert assessment of the images in the training data, and thus include ground truth values for the lung perfusion index. The adjustment of the parameters of the second neural network NN2 may be performed in accordance with the backpropagation techniques described above. The resulting trained second neural network NN2 is trained such that when provided with a new image from the first imaging modality, e.g. from the CT imaging modality, or from the second imaging modality, e.g. the X-ray projection imaging modality, the second neural network NN2 outputs the predicted value for the lung perfusion index. The second neural network NN2 may similarly be trained to predict the lung perfusion index from images from another type of imaging modality, such as for example an MRI imaging modality. The training of the second neural network NN2 is then performed in a similar manner. In general, the second neural NN2 network may be trained to generate the normalised measurement value 150_{1..i} for medical images from a single type of imaging modality, or for medical images from multiple types of imaging modalities. Independent neural networks may also be trained to generate the normalised measurement value 150_{1..i} for different types of imaging modalities.

In another example, a computer program product is provided. The computer program product comprises instructions which when executed by one or more processors, cause the one or more processors to carry out a method of compensating for differences in medical images, the method comprising:
receiving S110 image data comprising a temporal series of medical images 110_{1..i}, the temporal series including one or more medical images generated by a first type of imaging modality 120, and one or more medical images generated by a second type of imaging modality 130, 130', the first type of imaging modality being different to the second type of imaging modality;
generating S120a, S120b, from the temporal series of medical images 110_{1..i}, a normalised temporal series of medical images 140_{1..i}, or one or more normalised measurement values 150_{1..j} representing a region of interest in the temporal series of medical images 110_{1..i}; and
outputting S130a, S130a', S130b the normalised temporal series of medical images 140_{1..i} and/or one or more measurement values derived from the normalised temporal series of medical images 140_{1..i}, or outputting the one or more normalised measurement values 150_{1..j}, respectively.

In another example, a system 200 for compensating for differences in medical images, is provided. The system comprises one or more processors 210 configured to:
receive S110 image data comprising a temporal series of medical images 110_{1..i}, the temporal series including one or more medical images generated by a first type of imaging modality 120, and one or more medical images generated by a second type of imaging modality 130, 130', the first type of imaging modality being different to the second type of imaging modality;
generate S120a, S120b, from the temporal series of medical images 110_{1..i}, a normalised temporal series of medical images 140_{1..i}, or one or more normalised measurement values 150_{1..j} representing a region of interest in the temporal series of medical images 110_{1..i}; and
output S130a, S130a', S130b the normalised temporal series of medical images 140_{1..i} and/or one or more measurement values derived from the normalised temporal series of medical images 140_{1..i}, or outputting the one or more normalised measurement values 150_{1..j}, respectively.

An example of the system 200 is illustrated in Fig. 2. It is noted that the system 200 may also include one or more of: a plurality of medical imaging systems for generating the image data that is received in the operation S110, such as for example the projection X-ray imaging system 220 illustrated in Fig. 2, and a CT imaging system, or an MRI imaging system; a display device such as the monitor 250, a tablet, and so forth, for displaying the outputted data, such as the normalised temporal series of medical images 140_{1..i}, the one or more measurement values derived from the normalised temporal series of medical images 140_{1..i}, the one or more normalised measurement values 150_{1..j}, and so forth; a patient bed 260; and a user input device (not illustrated in Fig. 2) for receiving user input in relation to operations carried-out by the system, such as a keyboard, a mouse, a touchscreen, and so forth.

The above examples are to be understood as illustrative of the present disclosure, and not restrictive. Further examples are also contemplated. For instance, the examples described in relation to computer-implemented methods, may also be provided by the computer program product, or by the computer-readable storage medium, or by the system 200, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of compensating for differences in medical images, the method comprising:
receiving (S110) image data comprising a temporal series of medical images (1 10_{1..i}), the temporal series including one or more medical images generated by a first type of imaging modality (120), and one or more medical images generated by a second type of imaging modality (130, 130'), the first type of imaging modality being different to the second type of imaging modality;
generating (S120a, S120b), from the temporal series of medical images (1 10_{1..i}), a normalised temporal series of medical images (140_{1..i}), or one or more normalised measurement values (150_{1..j}) representing a region of interest in the temporal series of medical images (1 10_{1..i}); and
outputting (S130a, S130a', S130b) the normalised temporal series of medical images (140_{1..i}) and/or one or more measurement values derived from the normalised temporal series of medical images (140_{1..i}), or outputting the one or more normalised measurement values (150_{1..j}), respectively.

2. The computer-implemented method according to claim 1, wherein the generating comprises generating (S120a) a normalised temporal series of medical images (140_{1..i}) from the temporal series of medical images (110_{i..i}); and
wherein the outputting comprises outputting (S130a, S130a') the normalised temporal series of medical images (140_{1..i}) and/or one or more measurement values derived from the normalised temporal series of medical images (140_{1..i}); and
wherein the normalised temporal series of medical images (140_{1..i}) represent images generated by a common type of imaging modality.

3. The computer-implemented method according to claim 2, wherein the first type of imaging modality (120) is a projection imaging modality and the second type of imaging modality (130, 130') is a volumetric imaging modality; and wherein the common type of imaging modality is the projection imaging modality; and
wherein the normalised temporal series of medical images (140_{1..i}) is generated by providing the normalised temporal series of medical images as a combination of the one or more medical images from the temporal series (110_{1..i}) that are generated by the first type of imaging modality (120) and one or more projection images, and wherein the one or more projection images are provided by projecting the one or more medical images generated by the second type of imaging modality (130, 130') such that the one or more projected images correspond to one or more of the medical images generated by the first type of imaging modality (120).

4. The computer-implemented method according to claim 3, wherein the projecting comprises projecting the one or more medical images generated by the second type of imaging modality onto a virtual detector (240ᵥ) using a virtual source (230ᵥ).

5. The computer-implemented method according to claim 3 or claim 4, wherein the projecting is based on a known relative positioning between the virtual source, the virtual detector, and a subject represented in the one or more medical images generated by the second type of imaging modality (120); and/or
wherein the projecting comprises adjusting a relative positioning between the virtual source, the virtual detector, and the one or more medical images generated by the second type of imaging modality (130, 130') such that a shape of one or more anatomical features in the projected one or more images corresponds to a shape of the one or more corresponding anatomical features in the one or more medical images generated by the first type of imaging modality (120).

6. The computer-implemented method according to any one of claims 2 - 5, wherein the generating (S120a) a normalised temporal series of medical images (140_{i..i}) comprises:
warping one or more of the images in the normalised temporal series of medical images (140_{1..i}) such that a shape of one or more anatomical features (160) in the warped one or more images (140'_{1, 2}) corresponds to a shape of the one or more anatomical features (160) in a reference image (170).

7. The computer-implemented method according to claim 6, wherein the warping is based on a mapping between a plurality of corresponding landmarks (180_{i..j}) represented in both the warped image and the reference image (170).

8. The computer-implemented method according to any one of claims 2 - 7, wherein the generating (S120a) a normalised temporal series of medical images (140_{i..i}) comprises:
adjusting an intensity of the images in the normalised temporal series of medical images (140_{1..i}) based on an intensity at one or more positions in a reference image (170);
and/or
adjusting an intensity of the images in the normalised temporal series of medical images (140_{1..i}) using an image style transfer algorithm.

9. The computer-implemented method according to any one of claims 2 - 8, wherein the method further comprises:
receiving input defining a region of interest in the received temporal series of medical images (110_{1..i}); and
suppressing one or more image features outside the region of interest, or within the region of interest, in the normalised temporal series of medical images (140_{1..i}).

10. The computer-implemented method according to claim 9, wherein the region of interest is defined in the reference image (170), and wherein the method further comprises:
mapping an outline of the region of interest from the reference image to the images in the normalised temporal series of medical images (140_{1..i}); and
adjusting image intensity values in the images in the normalised temporal series of medical images (140_{1..i}) outside the mapped outline, or within the mapped outline, respectively, to suppress the one or more image features outside the region of interest.

11. The computer-implemented method according to any one of claims 6 - 10, wherein the reference image (170) is provided by: an image from the received temporal series of medical images (1 10_{1..i}), or an image from the normalised temporal series of medical images (140_{1..i}), or an atlas image.

12. The computer-implemented method according to claim 2, wherein the first type of imaging modality (120) is a projection imaging modality and the second type of imaging modality (130, 130') is a volumetric imaging modality; and wherein the common type of imaging modality is the projection imaging modality; and
wherein the normalised temporal series of medical images (140_{1..i}) is generated by providing the normalised temporal series of medical images as a combination of the one or more medical images from the temporal series (110_{1..i}) that are generated by the first type of imaging modality (120) and one or more projection images, and wherein the one or more projection images are provided by inputting the one or more medical images generated by the second type of imaging modality (130, 130') into a neural network (NN1); and
wherein the neural network (NN1) is trained to generate a projection image corresponding to the first type of imaging modality (120) for each of the inputted images.

13. The computer-implemented method according to claim 12, wherein the neural network (NN1) is trained to generate a projection image corresponding to the first type of imaging modality (120) for each of the inputted images by:
receiving training data, the training data comprising a plurality of volumetric training images representing the region of interest, the volumetric training images being generated by the second type of imaging modality (130, 130');
receiving ground truth data, the ground truth data comprising, for each of the volumetric training images, a corresponding ground truth projection image generated by the first type of imaging modality (120);
inputting the training data into the neural network (NN1); and
for each of a plurality of the inputted volumetric training images:
predicting, using the neural network (NN1), a corresponding projection image;
adjusting parameters of the neural network (NN1) based on a difference between the predicted projection image and the ground truth projection image; and
repeating the predicting and the adjusting, until a stopping criterion is met.

14. The computer-implemented method according to claim 1, wherein the generating comprises generating (S120b), from the temporal series of medical images (1 10_{1..i}), one or more normalised measurement values (150_{1..i}) representing a region of interest in the temporal series of medical images (110_{1..i}); and
wherein the outputting comprises outputting (S130b) the one or more normalised measurement values (150_{1..i}); and
wherein the one or more normalised measurement values (150_{1..i}) representing the region of interest are generated by:
inputting the temporal series of medical images (110_{1..i}) into a neural network (NN2); and
wherein the neural network (NN2) is trained to generate the normalised measurement value (150_{1..i}) representing the region of interest for each of the inputted images (1 10_{1..i}).

15. The computer-implemented method according to claim 14, wherein the neural network (NN2) is trained to generate the normalised measurement value (150_{1..i}) representing the region of interest for each of the inputted images by:
receiving training data, the training data comprising a plurality of training images representing the region of interest, the training images including a plurality of images generated by the first type of imaging modality (120) and a plurality of images generated by the second type of imaging modality (130, 130');
receiving ground truth data, the ground truth data comprising, for each of the training images, a corresponding ground truth value of the normalised measurement value representing the region of interest;
inputting the training data into the neural network (NN2); and
for each of a plurality of the inputted training images:
predicting, using the neural network (NN2), a value of the normalised measurement value representing the region of interest;
adjusting parameters of the neural network (NN2) based on a difference between the predicted value of the normalised measurement value and the corresponding ground truth value; and
repeating the predicting and the adjusting, until a stopping criterion is met.
